(19) Europäisches Patentamt
European Patent Office
Office européen des brevets

(11) Veröffentlichungsnummer: **0 290 863 A2**

(12) **EUROPÄISCHE PATENTANMELDUNG**

(21) Anmeldenummer: **88106712.8**

(22) Anmeldetag: **27.04.88**

(51) Int. Cl.4: **C07D 413/04 , C07D 265/36 , A01N 43/84**

Die Bezeichnung der Erfindung wurde geändert (Richtlinien für die Prüfung im EPA, A-III, 7.3).

(30) Priorität: **09.05.87 JP 111696/87
02.02.88 JP 21359/88**

(43) Veröffentlichungstag der Anmeldung: **17.11.88 Patentblatt 88/46**

(84) Benannte Vertragsstaaten: **BE CH DE FR GB IT LI NL**

(71) Anmelder: **NIHON TOKUSHU NOYAKU SEIZO K.K.
Itohpia Nihonbashi Honcho Building 7-1,
Nihonbashi Honcho 2-chome
Chuo-ku Tokyo 103(JP)**

(72) Erfinder: **Toyohiko, Kume
6-7-8, Asahigaoka
Hino-shi Tokyo(JP)**
Erfinder: **Toshio, Goto
3454-21, Honmachida
Machida-shi Tokyo(JP)**
Erfinder: **Atsumi, Kamochi
2-24-10, Higashi-Toyoda
Hino-shi Tokyo(JP)**
Erfinder: **Shigeki, Yagi
1-30-7, Izumi Suginami-ku
Tokyo(JP)**

(74) Vertreter: **Ernst, Hilmar, Dr. et al
Bayer AG Konzernverwaltung RP
Patentabteilung
D-5090 Leverkusen, Bayerwerk(DE)**

(54) **Benzoxazin-Derivat, Verfahren und Zwischenprodukte zu seiner Herstellung und seine Verwendung als Herbizid.**

(57) Die Erfindung betrifft das neue Benzoxazin der Formel (I)

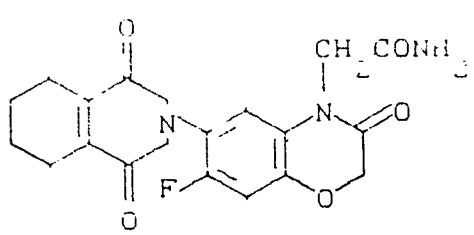

(I)

,

seine Herstellung und Verwendung als Herbizid und die neuen Zwischenprodukte zu seiner Herstellung der Formel

EP 0 290 863 A2

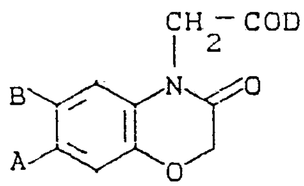

in der
A Wasserstoff oder Fluor ist,
B Wasserstoff oder Nitro ist und
D Amino, Hydroxy oder Ethoxy ist.

## Neue Benzoxazine

Die vorliegende Erfindung betrifft ein neues Benzoxazin, Verfahren und neue Zwischenprodukte zu seiner Herstellung und seine Verwendung als Herbizid.

Es wurde bereits offenbart, daß bestimmte Tetrahydrophthalimide, wie beispielsweise 2-(7-Fluoro-2H-1,4-benzoxazin-3(4-)-on-6-yl)-4,5,6,7-tetrahydro-2H-isoindol-1,3-dion, herbizide Aktivitäten besitzen (siehe die EP-A-0 170 191).

Nunmehr wurde das neue 2-[4-Carbamoylmethyl-7-fluoro-2H-1,4-benzoxazin-3(4H)-on-6-yl]-4,5,6,7-tetrahydro-2H-isoindol-1,3-dion der Formel (I)

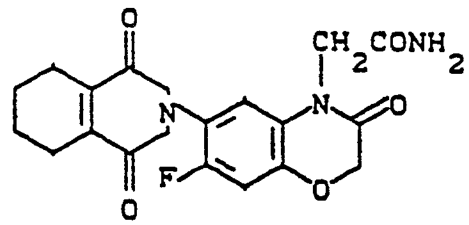

(I)

gefunden.

Die Verbindung der Formel (I) wird durch ein Verfahren erhalten, bei dem

a) 4-Carbamoylmethyl-7-fluoro-6-amino-2H-1,4-benzoxazin-3(4H)-on der Formel (II)

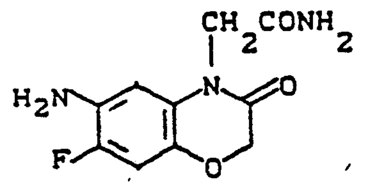

(II)

mit 3,4,5,6-Tetrahydrophthalsäureanhydrid in Gegenwart inerter Lösungsmittel umgesetzt wird.

Das neue Benzoxazin der Formel (I) zeigt potente herbizide Eigenschaften. Überraschenderweise zeigt das erfindungsgemäße Benzoxazin eine größere herbizide Aktivität als diejenigen, die aus dem oben genannten Stand der Technik bekannt sind, und eine günstige Verträglichkeit mit Nutzpflanzen, ohne Phytotoxizität.

Das Verfahren a) kann durch die folgende Gleichung dargestellt werden:

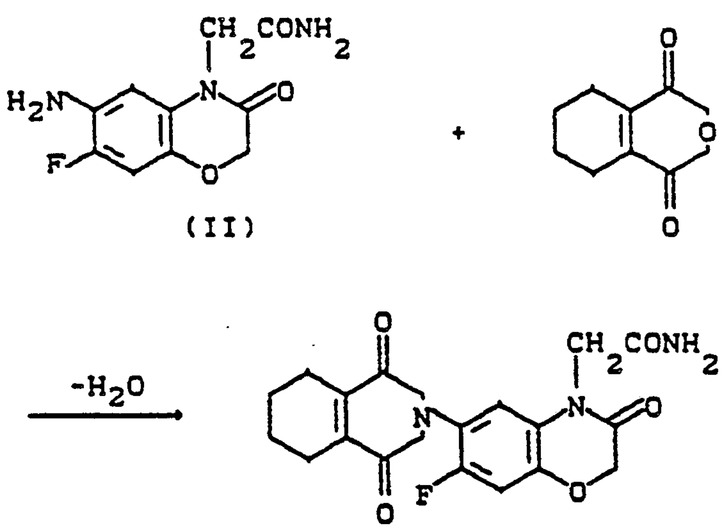


(II)

(I)

Wie in den hiernach beschriebenen Beispielen gezeigt wird, kann die Verbindung der Formel (II) in dem

Verfahren a) durch ein Verfahren hergestellt werden, bei dem

b) 4-Carbamoylmethyl-7-fluoro-6-nitro-2H-1,4-benzoxazin-3(4H)-on der Formel (III)

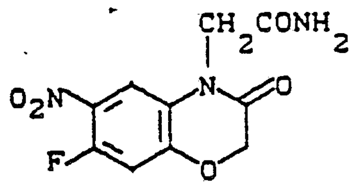

(III)

in Gegenwart inerter Lösungsmittel und in Gegenwart einer katalytischen Menge Palladium-Kohlenstoff reduziert wird.

In dem Verfahren b) findet eine katalytische Reduktion unter Verwendung einer katalytischen Menge Palladium-Kohlenstoff leicht und in einfacher Weise statt.

Weiterhin ist auch ein herkömmliches Reduktionsverfahren unter Verwendung von Eisen in Gegenwart einer Säure wie Essigsäure anwendbar.

Die Verbindung der obigen Formel (III) in dem Verfahren b) ist eine neue Verbindung, und sie kann, wie in den hiernach beschriebenen Beispielen gezeigt wird, durch ein Verfahren hergestellt werden, bei dem

c) 4-Carbamoylmethyl-7-fluoro-2H-1,4-benzoxazin-3(4H)-on der Formel (IV)

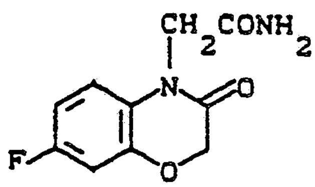

(IV)

mit konzentrierter Salpetersäure in Gegenwart von Schwefelsäure umgesetzt wird, oder

d) 4-Carboxymethyl-7-fluoro-6-nitro-2H-1,4-benzoxazin-3(4H)-on der Formel (V)

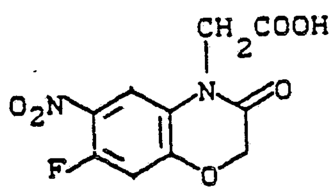

(V)

mit Thionylchlorid in Gegenwart inerter Lösungsmittel umgesetzt wird und das resultierende Produkt weiter mit Ammoniak umgesetzt wird.

Das Verfahren c) kann entsprechend der in "Synthetic Organic Chemistry". Seiten 747-749, oder in der Beschriebung der EP-OS 0 170 191 beschriebenen Nitrierungsreaktion durchgeführt werden.

Das Verfahren d) kann entsprechend den hiernach angegebenen Verfahren g) und Verfahren e) durchgeführt werden.

Die Verbindung der obigen Formel (IV) in dem Verfahren c) ist eine neue Verbindung, und sie kann, beispielsweise, durch ein Verfahren hergestellt werden, bei dem

e) 4-Chlorocarbonylmethyl-7-fluoro-2H-1,4-benzoxazin-3(4H)-on der Formel (VI)

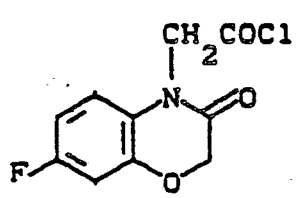

(VI)

mit Ammoniak in Gegenwart inerter Lösungsmittel umgesetzt wird, oder

f) 4-Ethoxycarbonylmethyl-7-fluoro-2H-1,4-benzoxazin-3(4H)-on der Formel (VII)

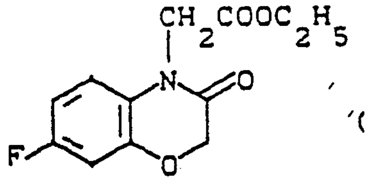

(VII)

mit Ammoniak in Gegenwart inerter Lösungsmittel umgesetzt wird.

Die Verbindung der obigen Formel (VI) in dem Verfahren e) ist eine neue Verbindung, und sie kann, wie in den hiernach beschriebenen Beispielen gezeigt wird, durch ein Verfahren hergestellt werden, bei dem

g) 4-Carboxymethyl-7-fluoro-2H-1,4-benzoxazin-3(4H)-on der Formel (VIII)

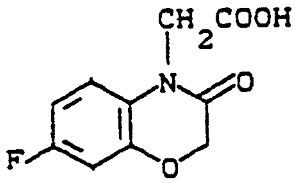

(VIII)

mit Thionylchlorid in Gegenwart inerter Lösungsmittel umgesetzt wird.

Bei der Durchführung des vorstehenden Verfahrens g) und anschließend des Verfahrens e) zur Synthetisierung der Verbindung (IV) in dem vorstehenden Verfahren c) kann die Verbindung der Formel (IV) in einfacher Weise dadurch hergestellt werden, daß die Verbindung der Formel (VIII) mit Thionylchlorid umgesetzt wird und das resultierende Produkt anschließend, statt einer Abtrennung der Verbindung der Formel (VI), mit Ammoniak umgesetzt wird, wie in dem hiernach beschriebenen Beispiel gezeigt wird.

Das Verfahren f) kann entsprechend der Beschreibung in "Synthetic Organic Chemistry", Seiten 565-569, R. B. Wagner, H.D. Zook, herausgegeben 1953 von John Wiley & Sons Inc., durchgeführt werden.

Die Verbindung der obigen Formel (VIII) in dem Verfahren g) ist eine neue Verlbindung, und sie kann, wie in den hiernach beschriebenen Beispielen gezeigt wird, durch ein Verfahren hergestellt werden, bei dem

h) 4-Ethoxycarbonylmethyl-7-fluoro-2H-1,4-benzoxazin-3(4H)-on der vorstehenden Formel (VII) in Gegenwart inerter Lösungsmittel hydrolysiert wird.

Die Verbindung der Formel (VII) in dem Verfahren f) und dem Verfahren h) kann, wie in dem hiernach beschriebenen Beispiel gezeigt wird, durch ein Verfahren hergestellt werden, bei dem

i) 7-[4-(Ethoxycarbonylmethyl)-2H-1,4-benzoxazin-3(4H)-on]diazonium-tetrafluoroborat der Formel (IX)

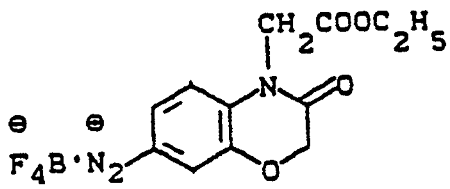

(IX)

in Gegenwart inerter Lösungsmittel thermisch zersetzt wird.

Das Verfahren i) kann entsprechend der in "Organic Reactions", Nr. 5, Seiten 199-206, beschriebenen Schiemann-Reaktion durchgeführt werden.

Die Verbindung der obigen Formel (IX) in dem Verfahren i) kann, wie in dem hiernach beschriebenen Beispiel gezeigt wird, durch ein Verfahren hergestellt werden, bei dem

j) 7-Amino-4-ethoxycarbonylmethyl-2H-1,4-benzoxazin-3(4H)-on der Formel (X)

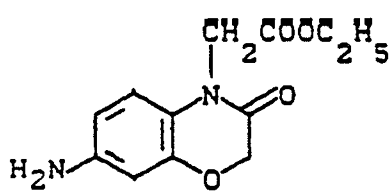

(X

mit Natriumnitrit und Tetrafluoroborsäure in Gegenwart inerter Lösungsmittel umgesetzt wird.

Die Verbindung der obigen Formel (X) in dem Verfahren j) kann, wie in dem hiernach beschriebenen Bezugsbeispiel gezeigt wird, in einfacher Weise durch ein Verfahren hergestellt werden, bei dem

k) 4-Ethoxycarbonylmethyl-7-nitro-2H-1,4-benzoxazin-3(4H)-on der Formel (XI)

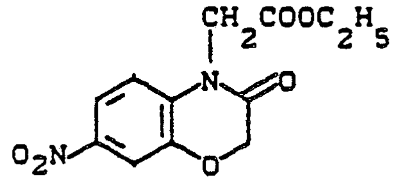

(XI

in Gegenwart inerter Lösungsmittel reduziert wird.

Die Verbindung der obigen Formel (XI) kann, wie in dem hiernach beschriebenen Beispiel gezeigt wird, in einfacher Weise durch ein Verfahren hergestellt werden, bei dem

l) 7-Nitro-2H-1,4-benzoxazin-3(4-)-on der Formel (XII)

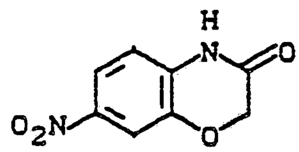

(XII)

mit dem Ethylester einer α-Halogenoessigsäure, vorzugsweise mit Ethyl-bromoacetat umgesetzt wird, oder

m) 2-Amino-5-nitrophenol der Formel (XIII)

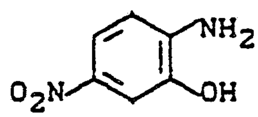

(XIII)

mit Ethyl-bromoacetat umgesetzt wird.

Das vorstehende Verfahren l) kann entsprechend dem in "Yakugaku Zasshi (Pharmacological Journal)", Band 86 (Nr. 3), 1966, Seiten 209-213, beschriebenen Verfahren durchgeführt werden.

Das Verfahren m) kann durch Umsetzung von 2 Äquivalenten Ethyl-bromoacetat pro Äquivalent der Verbindung der Formel (XIII) in Gegenwart eines Überschusses einer Base, beispielsweise Kaliumcarbonat, durchgeführt werden.

Die Verbindung der Formel (XII) in dem Verfahren 1) kann aus 2-Amino-5-nitrophenol hergestellt werden, wie in "Synthesis" 1982, Seiten 986-987, beschrieben ist.

Die Verbindung der obigen Formel (V) in dem Verfahren d) ist eine neue Verbindung, und sie kann, wie in dem hiernach beschriebenen Beispiel gezeigt wird, durch ein Verfahren hergestellt werden, bei dem

n) 4-Ethoxycarbonylmethyl-7-fluoro-6-nitro-2H-1,4-benzoxazin-3(4H)-on der Formel (XIV)

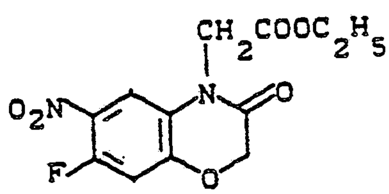

(XIV)

in Gegenwart inerter Lösungsmittel hydrolysiert wird, oder

o) 4-Carboxymethyl-7-fluoro-2H-1,4-benzoxazin-3(4H)-on der Formel (VIII) mit konzentrierter Salpetersäure in Gegenwart von Schwefelsäure umgesetzt wird.

Die Verbindung der Formel (XIV) in dem Verfahren n) ist eine neue Verbindung, und sie kann durch ein Verfahren hergestellt werden, bei dem

p) 4-Ethoxycarbonylmethyl-7-fluoro-2H-1,4-benzoxazin-3(4H)-on der Formel (VII) mit konzentrierter Salpetersäure in Gegenwart von Schwefelsäure umgesetzt wird.

Die Verbindungen der Formeln (III), (IV), (V), (VII), (VIII) und (XIV) sind neu und können in der folgenden Formel zusammengefaßt werden

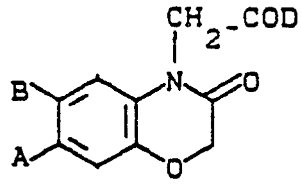

in der
A Wasserstoff oder Fluor ist,
B Wasserstoff oder Nitro ist und
D Amino, Hydroxy oder Ethoxy ist.

Bei der Durchführung des Verfahrens a) können organische Säuren wie Essigsäure oder Propionsäure und dergleichen als Verdünnungsmittel verwendet werden, und das Verfahren kann bei einer Temperatur von Raumtemperatur bis zum Siedepunkt einer zu verwendenden organischen Säure durchgeführt werden.

Vorzugsweise wird die Reaktion bei normalen Drücken durchgeführt, wenngleich ein höherer oder niedrigerer Druck ebenfalls angewandt werden kann.

Bei der Durchführung des Verfahrens a) werden beispielsweise etwa 1 bis 1,2 mol Tetrahydrophthalsäureanhydrid mit 1 mol der Verbindung der Formel (II) in Gegenwart des vorstehend genannten Verdünnungsmittels umgesetzt, wodurch die gewünschte Verbindung der Formel (I) erhalten wird.

Bei der Durchführung des Verfahrens b) können als geeignete Verdünnungsmittel alle Arten von inerten organischen Lösungsmitteln genannt werden.

Beispiele für die Verdünnungsmittel sind Wasser; aliphatische, alicyclische und aromatische Kohlenwasserstoffe (sie können chloriert sein, je nach den Umständen) wie Hexan, Cyclohexan, Petrolether, Ligroin, Benzol, Toluol, Xylol, Methylenchlorid, Chloroform, Kohlenstofftetrachlorid, Ethylenchlorid, Chlorobenzol; weiterhin Ether wie Diethylether, Methylethylether, Diisopropylether, Dibutylether, Dioxan, Tetrahydrofuran; Alkohole wie Methanol, Ethanol, Isopropanol; Ester wie Ethylacetat, Amylacetat; Säureamide wie Dimethylformamid, Dimethylacetamid; Sulfone und Sulfoxide wie Dimethylsulfoxid, Sulfolan; und organische Säuren wie Essigsäure und dergleichen.

Das Verfahren b) kann im wesentlichen innerhalb eines breiten Temperaturbereichs durchgeführt werden. Im allgemeinen kann es bei einer Temperatur von etwa 0 °C bis etwa 150 °C, vorzugsweise von etwa 20 °C bis etwa 100 °C, durchgeführt werden. Es wird bevorzugt, die Reaktion unter normalem Druck durchzuführen, wenngleich auch ein höherer oder niedrigerer Druck angewandt werden kann.

Bei der Durchführung der Verfahren c), o) und p) können als geeignete Verdünnungsmittel Schwefelsäure, Essigsäure oder Essigsäureanhydrid und dergleichen genannt werden. Diese Verfahren können im allgemeinen bei einer Temperatur von etwa -20 °C bis etwa 30 °C, vorzugsweise von etwa 0 °C bis etwa 20 °C, durchgeführt werden.

Bei der Durchführung der Verfahren d), e) und g) können als geeignete Verdünnungsmittel alle Arten von inerten organischen Lösungsmitteln genannt werden.

Beispiele für die Verdünnungsmittel sind Wasser; aliphatische, alicyclische und aromatische Kohlenwasserstoffe (sie können chloriert sein, je nach den Umständen) wie Hexan, Cyclohexan, Petrolether, Ligroin, Benzol, Toluol, Xylol, Methylenchlorid, Chloroform, Kohlenstofftetrachlorid, Ethylenchlorid, Chlorobenzol; weiterhin Ether wie Diethylether, Methylethylether, Diisopropylether, Dibutylether, Dioxan, Tetrahydrofuran; Ketone wie Aceton, Methylethylketon, Methylisopropylketon, Methylisobutylketon; Nitrile wie Acetonitril, Propionitril, Acrylnitril; Alkohole wie Methanol; Ester wie Ethylacetat, Amylacetat; Säureamide wie Dimethylformamid, Dimethylacetamid; Sulfone und Sulfoxide wie Dimethylsulfoxid, Sulfolan; und Basen wie Pyridin und dergleichen.

Die Verfahren d), e) und g) können im wesentlichen innerhalb eines breiten Temperaturbereichs durchgeführt werden. Sie können im allgemeinen bei einer Temperatur von etwa -20 °C bis etwa 150 °C, vorzugsweise von etwa 0 °C bis etwa 100 °C, durchgeführt werden. Die Reaktion wird vorzugsweise unter normalem Druck durchgeführt, wenngleich auch ein höherer oder niedrigerer Druck angewandt werden kann.

Bei der Durchführung des Verfahrens f) können als geeignete Verdünnungsmittel alle Arten von inerten organischen Lösungsmittel genannt werden.

Beispiele für die Verdünnungsmittel sind Wasser, aliphatische, alicyclische und aromatische Kohlenwasserstoffe (sie können chloriert sein, je nach den Umständen) wie Hexan, Cyclohexan, Petrolether, Ligroin, Benzol, Toluol, Xylol, Chlorobenzol; weiterhin Ether wie Diethylether, Methylethylether, Diisopropylether, Dibutylether, Dioxan, Tetrahydrofuran; Ketone wie Aceton, Methylethylketon, Methylisopropylketon, Methylisobutylketon; Alkohole wie Methanol, Ethanol, Isopropanol, Butanol, Ethylenglycol und dergleichen.

Das Verfahren f) kann im wesentlichen innerhalb eines breiten Temperaturbereichs durchgeführt werden. Es kann im allgemeinen bei einer Temperatur von etwa 50 °C bis etwa 200 °C, vorzugsweise von etwa 80 °C bis etwa 150 °C, durchgeführt werden. Die Reaktion kann unter normalem Druck durchgeführt werden, wenngleich auch ein höherer oder niedrigerer Druck angewandt werden kann.

Bei der Durchführung der Verfahren h) und n) können als geeignete Verdünnungsmittel alle Arten von inerten organischen Lösungsmitteln genannt werden.

Beispiele für die Verdünnungsmittel sind Wasser; aliphatische, alicyclische und aromatische Kohlenwasserstoffe (sie können chloriert sein, je nach den Umständen) wie Hexan, Cyclohexan, Petrolether, Ligroin, Benzol, Toluol, Xylol, Methylenchlorid, Chloroform, Kohlenstofftetrachlorid, Ethylenchlorid, Chlorobenzol; weiterhin Ether wie Diethylether, Methylethylether, Diisopropylether, Dibutylether, Dioxan, Tetrahydrofuran; Ketone wie Aceton, Methylethylketon, Methylisopropylketon, Methylisobutylketon; Nitrile wie Acetonitril, Propionitril, Acrylnitril; Alkohole wie Methanol, Ethanol, Isopropanol, Butanol, Ethylenglycol; Säureamide wie Dimethylformamid, Dimethylacetamid; Sulfone und Sulfoxide wie Dimethylsulfoxid, Sulfolan; und Basen wie Pyridin und dergleichen.

Die Verfahren h) und n) können im wesentlichen innerhalb eines breiten Temperaturbereichs durchgeführt werden. Sie können im allgemeinen bei einer Temperatur von etwa -20 °C bis etwa 150 °C, vorzugsweise von etwa -10 °C bis etwa 100 °C, durchgeführt werden. Es wird bevorzugt, die Reaktion unter normalem Druck durchzuführen, wenngleich auch ein höherer oder niedrigerer Druck angewandt werden kann.

Bei der Durchführung des Verfahrens i) können als geeignete Verdünnungsmittel alle Arten von inerten organischen Lösungsmitteln genannt werden, die einen Siedepunkt haben, der wenigstens dem thermischen Zersetzungspunkt (etwa 130 °C) der Verbindung der Formel (IX) entspricht.

Beispiele für solche Verdünnungsmittel sind aliphatische, alicyclische und aromatische Kohlenwasserstoffe (sie können chloriert sein, je nach den Umständen) wie Petrolether, Xylol, Chlorobenzol und dergleichen.

Das Verfahren i) kann im wesentlichen innerhalb eines breiten Temperaturbereichs durchgeführt werden. Es kann im allgemeinen bei einer Temperatur von etwa 125 °C bis etwa 180 °C, vorzugsweise von etwa 130 °C bis etwa 140 °C, durchgeführt werden. Es wird bevorzugt, die Reaktion unter normalem Druck durchzuführen, wenngleich auch ein höherer oder niedrigerer Druck angewandt werden kann.

Bei der Durchführung des Verfahrens j) können als geeignete Verdünnungsmittel Wasser, verdünnte Salzsäure, konzentrierte Salzsäure, Fluoroborsäure und dergleichen genannt werden.

Das Verfahren j) kann im wesentlichen innerhalb eines Temperaturbereichs durchgeführt werden. Es kann im allgemeinen bei einer Temperatur von etwa -30 °C bis etwa 30 °C, vorzugsweise von etwa -20 °C bis etwa 20 °C, durchgeführt werden. Es wird bevorzugt, die Reaktion unter normalem Druck durchzuführen, wenngleich auch ein höherer oder niedrigerer Druck angewandt werden kann.

Die erfindungsgemäßen aktiven Verbindungen können als Entlaubungsmittel, Abbrandmittel, Mittel zur Zerstörung breitblättriger Pflanzen und insbesondere als Unkrautvernichtungsmittel eingesetzt werden. Unter "Unkräutern" im weitesten Sinne sind alle Pflanzen zu verstehen, die an Stellen wachsen, wo sie nicht erwünscht sind. Ob die erfindungsgemäßen Substanzen als totale oder selektive Herbizide wirken, hängt im wesentlichen von der verwendeten Menge ab.

Die aktiven Verbindungen gemäß der Erfindung können beispielsweise in Verbindung mit den folgenden Pflanzen verwendet werden:

<u>Dikotyledonen-Unkräuter der Gattungen:</u>

Sinapis, Lepidium, Galium Stellaria, Matricaria, Anthemis, Galinsoga, Chenopodium, Urtica, Senecio, Amaranthus, Portulaca, Xanthium Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver und Centaurea.

Dikotyledonen-Kulturen der Gattungen:

Gossypium, Glycine, Beta, Daucus, Phaseolus, Pisum, Solanum, Linum, Ipomoea, Vicia, Nicotiana, Lycopersicon, Arachis, Brassica, Lactuca, Cucumis und Cucurbita.

Monokotyledonen-Unkräuter der Gattungen:

Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus und Apera.

Monokotyledonen-Kulturen der Gattungen:

Oryza, Zea, Triticum, Hordeum, Avena, Secale, Sorghum, Panicum, Saccharum, Ananas, Asparagus und Allium.

Die Anwendung der erfindungsgemäßen aktiven Verbindungen ist jedoch keineswegs auf diese Gattungen begrenzt, sondern umfaßt auch andere Pflanzen in gleicher Weise.

In Abhängigkeit von den Konzentrationen können die Verbindungen zur totalen Bekämpfung von Unkräutern, beispielsweise auf Industriegelände und Bahnkörpern sowie auf Wegen und Plätzen mit oder ohne Baumbestand, verwendet werden. Ebenso können die Verbindungen auch zur Unkrautbekämpfung in perennierenden Kulturen, beispielsweise Aufforstungen, Zierbaumpflanzungen, Obstgärten, Weinbergen und -gärten, Zitrushainen, Nußbaumpflanzungen, Bananenplantagen, Kaffeeplantagen, Teeplantagen, Gummiplantagen, Ölpalmenplantagen, Kakaoplantagen, Weichfruchtplantagen und Hopfenfeldern, so wie zur selektiven Unkrautbekämpfung in Jahreskulturen verwendet werden.

Die Wirkstoffe können in übliche Präparate wie Lösungen, Emulsionen, Suspensionen, Pulver, Stäubemittel, Schäume, Pasten, lösliche Pulver, Granulat, Aerosole, mit dem Wirkstoff imprägnierte natürliche und synthetische Materialien, sehr feine Kapseln in polymeren Substanzen, Beschichtungsmittel zum Aufbringen auf das Saatgut und Formulierungen, die in Verbindung mit Verbrennungseinrichtungen wie Räucherpatronen, Räucherdosen und Räucherschlangen eingesetzt werden, sowie ULV-Kaltvernebelungs-und Warmvernebelungspräparate umgewandelt werden.

Diese Präparate können in bekannter Weise hergestellt werden, beispielsweise durch Vermischen der Wirkstoffe mit Streckmitteln, d.h. flüssigen oder verflüssigten gasförmigen oder festen Verdünnungsmitteln oder Trägern, gegebenenfalls unter Verwendung von grenzflächenaktiven Mitteln, d.h. Emulgatoren und/oder Dispergiermitteln und/oder schaumbildenden Mitteln. Bei Verwendung von Wasser als Streckmittel können beispielsweise auch organische Lösungsmittel als Hilfslösungsmittel verwendet werden.

Als flüssige Verdünnungsmittel oder Träger eignen sich hauptsächlich aromatische Kohlenwasserstoffe wie Xylol, Toluol oder Alkylnaphthaline, chlorierte aromatische oder chlorierte aliphatische Kohlenwasserstoffe wie Chlorobenzole, Chloroethylene oder Methylenchlorid, aliphatische oder alicyclische Kohlenwasserstoffe wie Cyclohexan oder Paraffine, beispielsweise Mineralöl-Fraktionen, Alkohole wie Butanol oder Glycol sowie deren Ether und Ester, Ketone wie Aceton, Methylethylketon, Methylisobutylketon oder Cyclohexanon, oder stark polare Lösungsmittel wie Dimethylformamid oder Dimethylsulfoxid, sowie auch Wasser.

Unter verflüssigten gasförmigen Verdünnungsmitteln oder Trägern sind Flüssigkeiten zu verstehen, die bei normaler Temperatur und unter normalem Druck gasförmig sein würden, beispielsweise Aerosol-Triebmittel wie halogenierte Kohlenwasserstoffe sowie Butan, Propan, Stickstoff und Kohlenstoffdioxid.

Als feste Träger können gemahlene natürliche Minerale wie Kaoline, Tone, Talkum, Kreide, Quarz, Attapulgit, Montmorillonit oder Diatomeenerde, und gemahlene synthetische Minerale wie hochdisperse Kieselsäure, Aluminiumoxid und Silikate verwendet werden. Als feste Träger für Granulat können zerkleinertes und fraktioniertes natürliches Gesteinsmaterial wie Calcit, Marmor, Bimsstein, Sepiolith und Dolomit sowie synthetisches Granulat aus anorganischen und organischen Mehlen und Granulat aus organischem Material wie Sägemehl, Kokosnußschalen, Maiskolben und Tabakstengeln verwendet werden.

Als Emulgatoren und/oder Schaumbildner können nichtionische oder anionische Emulgatoren wie Polyoxyethylen-Fettsäureester, Polyoxyethylen-Fettalkoholether, beispielsweise Alkylarylpolyglycolether, Alkylsulfonate, Alkylsulfate, Arylsulfonate sowie Hydrolyseprodukte des Albumins verwendet werden. Zu den Dispergiermitteln zählen beispielsweise Ligninsulfit-Ablaugen und Methylcellulose.

Haftmittel wie Carboxymethylcellulose und natürliche und synthetische Polymerisate in Form von Pulver, Granulat oder Latices, z.B Gummi arabicum, Polyvinylalkohol und Polyvinylacetat, können in den Formulierungen verwendet werden.

Es ist möglich, farbgebende Stoffe wie anorganische Pigmente, beispielsweise Eisenoxid, Titanoxid und Preußischblau, und organische Farbstoffe wie Alizarin-Farbstoffe, Azo-Farbstoffe oder Metallphthalocyanin-Farbstoffe, und Spurennährstoffe wie Salze von Eisen, Mangan, Bor, Kupfer, Cobalt, Molybdän und Zink, zu verwenden.

Die Präparate enthalten im allgemeinen von 0,1 bis 95 Gewichts-%, vorzugsweise von 0,5 bis 90 Gewichts-% des Wirkstoffs.

Die Wirkstoffe gemäß der vorliegenden Erfindung können als solche oder in Form ihrer Präparate auch zur Unkrautbekämpfung als Mischungen mit anderen Herbiziden eingesetzt werden, wobei Fertigpräparate oder Tankmischungen möglich sind.

Mischungen mit anderen aktiven Verbindungen wie Herbiziden, Fungiziden, Insektiziden, Akariziden, Nematiziden, vogelabweisenden Mitteln, Pflanzennährstoffen sowie Mitteln zur Verbesserung der Bodenstruktur sind ebenfalls möglich.

Die Wirkstoffe können als solche, in form von Präparaten oder Gebrauchsformen, die daraus durch weitere Verdünnung hergestellt werden, eingesetzt werden, beispielsweise in Form gebrauchsfertiger Lösungen, Suspensionen, Emulsionen, Pulver, Pasten und als Granulat. Sie werden in üblicher Weise angewandt, beispielsweise durch Bewässern, Sprühen, Zerstäuben oder Streuen.

Die erfindungsgemäßen Wirkstoffe können vor oder nach dem Auflaufen der Pflanzen zur Anwendung gelangen.

Sie können auch vor der Einsaat in den Boden eingearbeitet werden. Insbesondere werden sie nach dem Auflaufen der Pflanzen angewandt.

Die eingesetzten Mengen der aktiven Verbindung können innerhalb eines Bereichs von erheblicher Breite variiert werden. Sie hängen wesentlich von der Art der angestrebten Wirkung ab. Im allgemeinen liegen die aufgewandten Mengen des Wirkstoffs zwischen etwa 0,0005 und etwa 5 kg/ha, vorzugsweise zwischen etwa 0,001 und etwa 3 kg/ha.

Die Herstellung und Verwendung der erfindungsgemäßen Verbindungen werden mit Hilfe der folgenden Beispiele veranschaulicht. Es ist jedoch darauf hinzuweisen, daß der Umfang der vorliegenden Erfindung nicht nur auf den technischen Inhalt der Beispiele beschränkt ist.

## Synthesebeispiele

### Beispiel 1

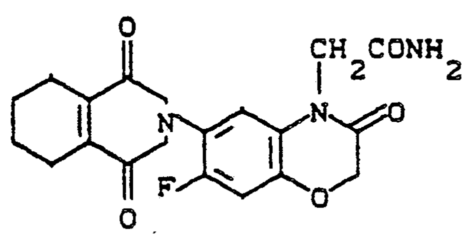

(I)

Eine Mischung aus 4-Carbamoylmethyl-7-fluoro-6-nitro-2H-1,4-benzoxazin-3(4H)-on (1,78 g), N,N-Dimethylformamid (50 ml) und 10-proz. Palladium-Kohlenstoff (0,3 g) wurde unter normalem Druck katalytisch reduziert. Nach Beendigung der Wasserstoff-Aufnahme wurde das resultierende Produkt abfiltriert, anschließend wurde das Lösungsmittel unter vermindertem Druck abdestilliert, dann wurden Essigsäure (30 ml) und Tetrahydrophthalsäureanhydrid (1 g) zugegeben, und die Mischung wurde 3 h zum Rückfluß erhitzt. Nach Beendigung der Reaktion wurde das Lösungsmittel unter vermindertem Druck abdestilliert, und Tetrahydrofuran (100 ml) wurde dem Rückstand zugesetzt. Die Flüssigkeit wurde heiß filtriert, und das Filtrat wurde unter vermindertem Druck destilliert, wonach ein zurückbleibender fester Stoff erhalten wurde.

Der feste Rückstand wurde mit Toluol gewaschen, und dann wurden Ethanol (150 ml) und Aktivkohle (0,5 g) zugegeben, und die Mischung wurde eine Minute erhitzt und dann filtriert. Das Filtrat wurde konzentriert und auf Raumtemperatur abgekühlt und dann abfiltriert. Das erhaltene Produkt wurde mit n-Hexan gewaschen, wonach das gewünschte 2-[4-Carbamoylmethyl-7-fluoro-2H-1,4-benzoxazin-3(4H)-on-6-yl]-4,5,6,7-tetrahydro-2H-isoindol-1,3-dion (0,5 g) erhalten wurde; Schmp. 283-286 °C.

Beispiel 2

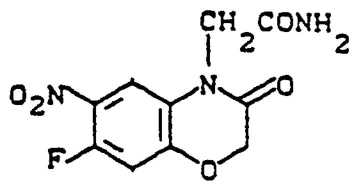

(III)

4-Carbamoylmethyl-7-fluoro-2H-1,4-benzoxazin-3(4H)-on (4,48 g) wurde auf eine Temperatur von 0 °C bis 5 °C abgehühlt und dann zu konzentrierter Schwefelsäure hinzugefügt, und die Mischung wurde gut gerührt. 98-proz. Salpetersäure (1,42 g) wurde bei einer Temperatur von 0 °C bis 5 °C tropfenweise dazugegeben. Nachdem die Flüssigkeit 1 h gerührt worden war, wurde sie in eiskaltes Wasser gegossen, und der erzeugte feste Stoff wurde abfiltriert, mit Wasser gewaschen und dann getrocknet, wonach das gewünschte 4-Carbamoylmethyl-7-fluoro-6-nitro-2H-1,4-benzoxazin-3(4H)-on (4,0 g) erhalten wurde; Schmp. 220-226 °C.

Beispiel 3

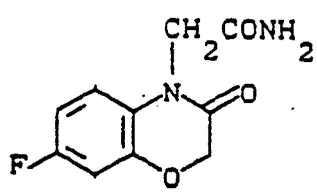

(IV)

Drei Tropfen Pyridin wurden zu einer Mischung von 4-Carboxymethyl-7-fluoro-2H-1,4-benzoxazin-3(4H)-on (2,25 g), Chloroform (18 g) und Thionylchlorid (3,6 g) hinzugefügt, und die Mischung wurde dann 5 h unter Rückfluß erhitzt. Nachdem die Lösung konzentriert worden war, wurde sie mit Tuluol (50 ml) vermischt. Nach dem Abdestillieren des Lösungsmittels wurde der Rückstand in Toluol (60 ml) gelöst, und Ammoniak-Gas wurde bei einer Temperatur von 10 °C bis 20 °C in die Lösung eingeleitet. Nach der Sättigung mit Ammoniak wurde die Lösung 1 h bei Raumtemperatur gerührt, und der feste Stoff wurde abfiltriert, dann mit Toluol, Natriumhydrogencarbonat-Lösung und Wasser, in dieser Reihenfolge, gewaschen und dann getrocknet, wonach das gewünschte 4-Carbamoylmethyl-7-fluoro-2H-1,4-benzoxazin-3(4H)-on (2,1 g) erhalten wurde; Schmp. 212-214 °C.

Beispiel 4

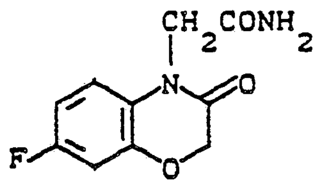

(IV)

Eine Mischung aus 4-Ethoxycarbonylmethyl-7-fluoro-2H-1,4-benzoxazin-3(4H)-on (1,27 g), Ethanol (20 ml) und 28-proz. Ammoniak in Wasser (10 g) wurde 2 h bei Raumtemperatur durchmischt und dann 3 h zum Rückfluß erhitzt. Nach dem Konzentrieren wurde die Mischung mit Wasser vermischt und filtriert. Das resultierende Produkt wurde mit Wasser und Ethanol, in dieser Reihenfolge, gewaschen und aus Methylisobutylketon umkristallisiert, wonach das gewünschte 4-Carbamoylmethyl-7-fluoro-2H-1,4-benzoxazin-3(4H)-on (0,45 g) erhalten wurde; Schmp. 216,5 bis 219 °C.

Beispiel 5

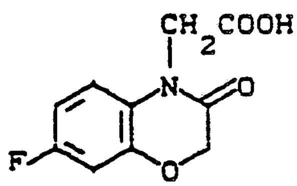

(VIII)

Natriumhydroxid (0,7 g) und Wasser (10 ml) wurden zu einer Mischung von 4-Ethoxycarbonylmethyl-7-fluoro-2H-1,4-benzoxazin-3(4H)-on (3,7 g) und Ethanol (20 ml) hinzugefügt, und die Mischung wurde 5 h zum Rückfluß erhitzt. Nach der Reaktion wurde die Lösung konzentriert, der Rückstand wurde in Wasser (50 ml) gelöst und mit Methylisobutylketon gewaschen, die wäßrige Schicht wurde mit Salzesäure angesäuert und mit Methylisobutylketon extrahiert, und der Extrakt wurde mit wasserfreiem Natriumsulfat getrocknet. Nach dem Abdestillieren des Lösungsmittels wurde der Rückstand mit einem Mischlösungsmittel aus Toluol-Hexan (1:5) versetzt und die Mischung wurde abfiltriert, wonach das gewünschte 4-Carboxymethyl-7-fluoro-2H-1,4-benzoxazin-3(4H)-on (3,2 g) erhalten wurde; Schmp. 183-184 °C.

Beispiel 6

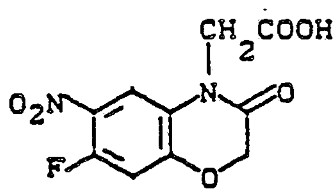

(V)

Natriumhydroxid (0,9 g) und Wasser (20 ml) wurden zu einer Mischung von 4-Ethoxycarbonylmethyl-7-fluoro-6-nitro-2H-1,4-benzoxazin-3(4H)-on (5,96 g) und 1,4-Dioxan (50 ml) hinzugefügt, und die Mischung wurde 1 d bei Raumtemperatur gerührt. Nach dem Abdestillieren des Lösungsmittels wurde der Rückstand mit Wasser (100 ml) vermischt, und die Mischung wurde filtriert. Das Filtrat wurde mit Salzsäure angesäuert und mit Methylisobutylketon (150 ml) extrahiert. Nachdem der Extrakt mit Wasser gewaschen worden war, wurde er mit wasserfreiem Natriumsulfat getrocknet, und dann wurde das Lösungsmittel abdestilliert, wonach das gewünschte 4-Carboxymethyl-7-fluoro-6-nitro-2H-1,4-benzoxazin-3(4H)-on (4,8 g) erhalten wurde; Schmp. 177-183 °C.

Beispiel 7

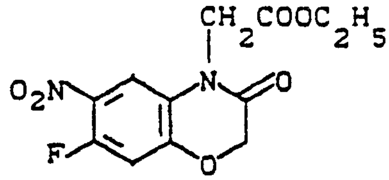

(XIV)

4-Ethoxycarbonylmethyl-7-fluoro-2H-1,4-benzoxazin-3(4H)-on (20 g) wurde bei einer Temperatur von 0 °C bis 5 °C zu konzentrierter Schwefelsäure hinzugefügt. Nachdem die Mischung kräftig (etwa 10 min) gerührt worden war, wurde 98-proz. Salpetersäure (5,4 g) tropfenweise hinzugefügt. Die Flüssigkeit wurde 1,5 h bei einer Temperatur von 0 °C bis 5 °C gerührt und dann in eiskaltes Wasser gegossen, und der entstandene feste Stoff wurde durch Filtration isoliert, gut mit Wasser gewaschen, bis die Waschflüssigkeit nicht mehr sauer reagierte, und dann getrocknet. Umkristallisation aus Ethanol ergab das gewünschte 4-Ethoxycarbonylmethyl-7-fluoro-6-nitro-2H-1,4-benzoxazin-3(4H)-on (21 g); Schmp. 79-81 °C.

Beispiel 8

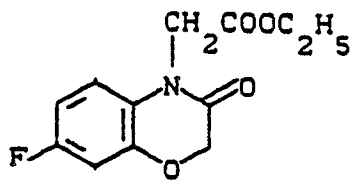

(VII)

7-[4-(Ethoxycarbonylmethyl)-2H-1,4-benzoxazin-3(4H)-on]diazonium-tetrafluoroborat (3,49 g) wurde etwa 5 min auf eine Temperatur von 130 °C bis 135 °C erhitzt, bis die Gasentwicklung aufhörte. Das Reaktionsprodukt wurde mit Tuluol (50 ml) vermischt, und die Mischung wurde unter vermindertem Druck zur Trockne eingedampft. Der Rückstand wurde mit Ethanol (100 ml) vermischt und mit Aktivkohle behandelt. Nach der Filtration wurde das Filtrat unter vermindertem Druck destilliert, und der verbleibende Rückstand (2,1 g) wurde aus Ethanol-Hexan umkristallisiert, wonach das gewünschte 4-Ethoxycarbonylmethyl-7-fluoro-2H-1,4-benzoxazin-3(4H)-on (0,6 g) erhalten wurde; Schmp. 90-91,5 °C.

Beispiel 9

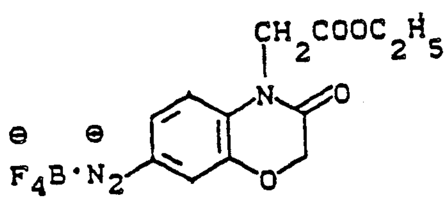

(IX)

36-proz. Salzsäure (24 g) wurde zu Wasser (200 ml) gegeben, und die Flüssigkeit wurde auf 10 °C abgekühlt. Dann wurde 7-Amino-4-ethoxycarbonylmethyl-2H-1,4-benzoxazin-3(4H)-on (20,2 g) hinzugefügt, und die Mischung wurde gerührt. Natriumnitrit (5,9 g), gelöst in Wasser (30 ml), wurde bei einer Temperatur von 0 °C bis 5 °C tropfenweise hinzugefügt; 30 min danach wurde 42-proz. Tetrafluoroborsäure (25 g) tropfenweise zu der Flüssigkeit hinzugefügt, und die Mischung wurde 1 h bei einer Temperatur von 0 °C bis 5 °C gerührt. Dann wurde die gewünschte Verbindung abfiltriert, zweimal mit 50 ml kaltem Wasser gewaschen und an der Luft getrocknet, wonach das gewünschte 7-[4-(Ethoxycarbonylmethyl)-2H-1,4-benzoxazin-3(4H)-on]diazonium-tetrafluoroborat (22 g) erhalten wurde; Schmp. 129-130 °C (Zers.).

Bezugsbeispiel 1

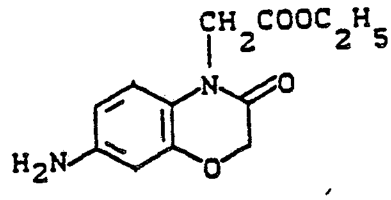

(X)

Eine Mischung aus 4-Ethoxycarbonylmethyl-7-nitro-2H-1,4-benzoxazin-3(4H)-on, 10-proz. Palladium-Kohlenstoff (3 g) und Ethanol (150 ml) wurde in einen Autoklaven gefüllt und unter den Bedingungen eines Wasserstoff-Drucks von 19,6 bar (20 kg/cm$^2$) und einer Temperatur von 60 °C bis 70 °C katalytisch hydriert. Tetrahydrofuran (150 ml) wurde hinzugefügt, dann wurde die Flüssigkeit filtriert, und das Filtrat wurde unter vermindertem Druck destilliert. Umkristallisation des resultierenden festen Stoffs aus Ethanol ergab das gewünschte 7-Amino-4-ethoxycarbonylmethyl-2H-1,4-benzoxazin-3(4H)-on (20 g); Schmp. 136-137 °C.

Bezugsbeispiel 2

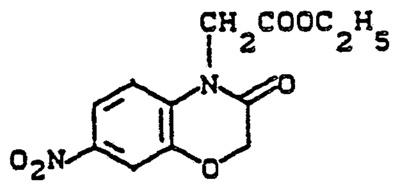

(XI)

Ethyl-bromoacetat (18,4 g) wurde bei einer Temperatur von 30 °C bis 40 °C tropfenweise zu einer Mischung aus 7-Nitro-2H-1,4-benzoxazin-3(4H)-on (19,4 g), Kaliumcarbonat (15,2 g) und Acetonitril (100 ml) hinzugegeben, und die Mischung wurde 3 h zum Rückfluß erhitzt. Nach beendeter Reaktion wurde die Flüssigkeit filtriert, und das Filtrat wurde destilliert. Die Umkristallisation des verbliebenen festen Stoffs aus Toluol-Hexan ergab das gewünschte 4-Ethoxycarbonylmethyl-7-nitro-2H-1,4-benzoxazin-3(4H)-on (20 g); Schmp. 120,5-121,5 °C.

Bezugsbeispiel 3

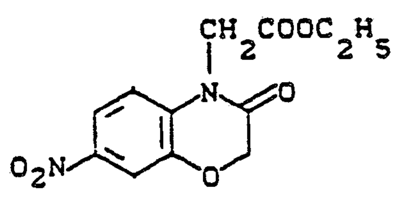

(XI)

Ethyl-bromoacetat (92 g) wurde bei einer Temperatur von 50 °C bis 60 °C tropfenweise zu einer Mischung aus 2-Amino-5-nitrophenol (38,5 g), Kaliumcarbonat (106,4 g) und N,N-Dimethylformamid (100 ml) hinzugefügt. Nachdem die Mischung 6 h bei einer Temperatur von 100 °C bis 110 °C gerührt worden war, wurde sie abgekühlt und in eiskaltes Wasser (500 g) gegossen. Die Flüssigkeit wurde zweimal mit Toluol (300 ml) extrahiert, die Extraktionsflüssigkeiten wurden vereinigt und mit Wasser, einer wäßrigen 5-proz. Kaliumhydroxid-Lösung und Wasser, in dieser Reihenfolge, gewaschen und dann mit wasserfreiem Natriumsulfat getrocknet. Danach wurde das Lösungsmittel abdestilliert. Die Umkristallisation des Rückstands aus Toluol-Hexan ergab das gewünschte 4-Ethoxycarbonylmethyl-7-nitro-2H-1,4-benzoxazin-3(4H)-on (30 g). Nach dem Destillieren der Mutterlauge und Umkristallisation aus Ethanol wurden weitere 1,3 g der gewünschten Verbindung erzeugt; Schmp. 120,5-121,5 °C.

In dem vorstehenden Bezugsbeispiel 3 wurden die Waschflüssigkeiten der wäßrigen 5-proz.

Kaliumhydroxid-Lösung mit konzentrierter Salzsäure angesäuert, und das Lösungsmittel wurde abdestilliert. Danach wurde der feste Rückstand durch Filtration isoliert, mit Wasser gewaschen und dann aus Ethanol umkristallisiert, wonach 7-Nitro-2H-1,4-benzoxazin-3(4H)-on (4,1 g) der Formel (XII) erhalten wurde.

ImFfolgenden wird eine Ausführungsform aufgezeigt, wie die Verbindung der vorerwähnten Formel (IV) aus der Verbindung der Formel (IX) als Ausgangsmaterial mit Hilfe der Verfahren i), h), g) und e) hergestellt wird.

Beispiel 10

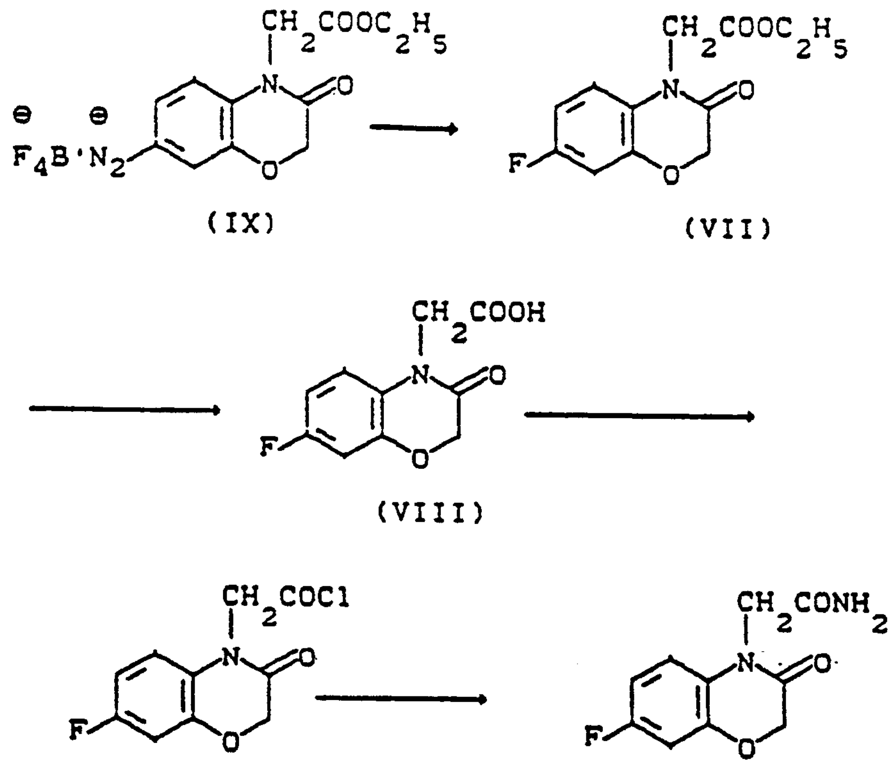

(IX) (VII) (VIII) (VI) (IV)

Eine Mischung aus dem Diazonium-Salz (6,4 g) der Formel (IX) und Xylol (160 ml) wurde 30 min zum Rückfluß erhitzt. Nach dem Entfernen des Lösungsmittels wurden dem Rückstand Ethanol (100 ml) und eine wäßrige Lösung von Natriumhydroxid (3,5 g), gelöst in Wasser (30 ml), zugesetzt, und die Flüssigkeit wurde 3 h zum Rückfluß erhitzt. Die Reaktionsmischung wurde unter vermindertem Druck zur Trockne konzentriert, der erhaltente Rückstand wurde in Wasser gelöst, Methylisobutylketon (30 ml) wurde zugemischt, und die wäßrige Schicht wurde mit Salzsäure angesäuert. Die salzsaure Reaktionsmischung wurde zweimal mit Methylisobutylketon (60 ml) extrahiert, und der Extrakt wurde mit wasserfreiem Natriumsulfat getrocknet. Das Lösungsmittel wurde abdestilliert, und zu dem resultierenden Rückstand (3,8 g) wurden Chloroform (3,8 g) Thionylchlorid (6,1 g) und Pyridin (6 Tropfen) hinzugefügt. Die Flüssigkeit wurde 5 h bei einer Temperatur von 50 °C bis 60 °C zum Rückfluß erhitzt. Das Lösungsmittel wurde konzentriert, Toluol (50 ml) wurde zugemischt, die Mischung wurde filtriert, das Filtrat wurde unter vermindertem Druck destilliert, um das verbleibende Thionylchlorid und Salzsäure abzudestillieren. Dann wurde Toluol (70 ml) zugemischt, und Ammoniak-Gas wurde bei einer Temperatur von 10 °C bis 20 °C eingeleitet. Danach ließ man die Reaktionsmischung unter Bedingungen der Sättigung mit dem Gas bei Raumtemperatur über Nacht stehen. Der feste Stoff wurde abfiltriert. Der feste Stoff wurde mit Wasser, einer Natriumhydrogencarbonat-Lösung und Wasser, in dieser Reihenfolge, gewaschen und dann getrocknet, wonach die gewünschte Verbindung der Formel (IV), 4-Carbamoylmethyl-7-fluoro-2H-1,4-benzoxazin-3(4H)-on (2,4 g) erhalten wurde; Schmp. 216-219 °C.

Biologische Tests

Vergleichs-Verbindung (E-1)

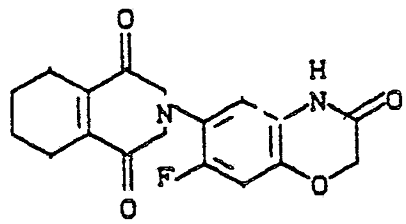

bekannt aus der EP-A-O 170 191 / Verbindung Nr. 7)

Beispiel A

Test gegen Unkräuter auf höher gelegenem Ackerboden durch Bodenbehandlung vor dem Auflaufen:

Herstellung eines Wirkstoff-Präparats

Träger: 5 Gewichtsteile Aceton;
Emulgator: 1 Gewichtsteil Polyglycolether.

Eine Formulierung einer aktiven Verbindung in Form eines emulgierbaren Konzentrats wurde erhalten durch Vermischen von 1 Gewichtsteil der aktiven Verbindung mit den oben angegebenen Mengen des Trägers und des emulgierenden Mittels. Eine vorher festgelegte Menge des Präparats wurde mit Wasser verdünnt.

Test-Verfahren

In einem Gewächshaus wurden Samen von Reis, Sojabohnen und Mais in Töpfe von 500 $cm^2$ Fläche, die mit Ackererde gefüllt waren, eingesät, und Erde, die Samen von grauem Amaranth (Amaranthus lividus L.) und Gänsefuß (Chenopodium album L.) enthielt, wurde über die Erde in den Töpfen in einer Tiefe von 1 cm gestreut.

Einen Tag nach der Einsaat wurde eine Test-Chemikalie in einer vorher festgelegten Konzentration gleichmäßig über die Oberflächenschicht des Bodens in jedem der Test-Töpfe gesprüht.

Vier Wochen nach dem Sprühen wurden die herbizide Wirkung und die Phytotoxizität gegenüber den Nutzpflanzen mit Hilfe der folgenden Skala von 0 bis 5 bewertet:

| Bewertung | Herbizide Wirkung (bewertet als Unkrautvernichtungs-Verhältnis, bezogen auf eine unbehandelte Fläche) |
|---|---|
| 5 | wenigstens 95 % (verdorrt) |
| 4 | wenigstens 80 %, jedoch weniger als 95 % |
| 3 | wenigstens 50 %, jedoch weniger als 80 % |
| 2 | wenigstens 30 %, jedoch weniger als 50 % |
| 1 | wenigstens 10 %, jedoch weniger als 30 % |
| 0 | weniger als 10 % (unwirksam) |

| Bewertung | Phytotoxizität gegenüber Nutzpflanzen (bewertet unter Bezug auf die unbehandelte Fläche) |
|---|---|
| 5 | wenigstens 90 % (Ausrottung) |
| 4 | wenigstens 50 %, jedoch weniger als 90 % |
| 3 | wenigstens 30 %, jedoch weniger als 50 % |
| 2 | wenigstens 10 %, jedoch weniger als 30 % |
| 1 | mehr als 0 %, jedoch weniger als 10 % |
| 0 | 0 % (keine Phytotoxizität) |

Test-Ergebnisse der Boden-Behandlung

| Aktive Verbindung | Dosis kg/ha | Herbizider Effekt AMALI | Herbizider Effekt CHEAL | Phytotoxischer Effekt ORYSD | Phytotoxischer Effekt GLXMA | Phytotoxischer Effekt ZEAMX |
|---|---|---|---|---|---|---|
| Verbindung der Formel (I) der vorliegenden Erfindung | 0,25 | 4 | 4 | 0 | 0 | 0 |
| bekannte Verbindung E-1 | 0,25 | 4 | 3 | 1 | 2 | 1 |

Anmerkung:

In der Tabelle bezeichen die Abkürzugen die nachstehenden Unkräuter und Nutzpflanzen:
AMALI: Amaranthus lividus L.
CHEAL: Chenopodium album L.
ORYSD: Oryza sativa L.
GLXMA: Glycine max. L.
ZEAMX: Zea mays L.
Verbindung der Formel (I):

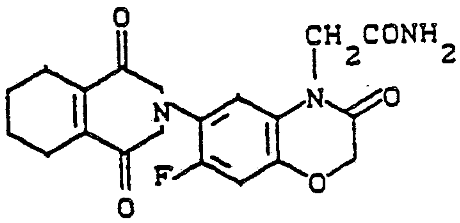

(I)

bekannte Verbindung (E-1):

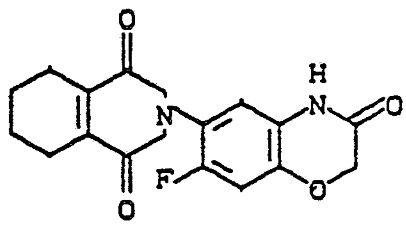

Beispiel B

Test gegen Unkräuter auf höher gelegenem Ackerboden durch Laub-Behandlung:

In einem Gewächshaus wurden Samen von Reis, Sojabohnen und Mais in Töpfe von 500 $cm^2$ Fläche, die mit Ackererde gefüllt waren, eingesät, und Erde, die Samen von grauem Amaranth (Amaranthus lividus L.) und Gänsefuß (Chenopodium album L.) enthielt, wurde über die Erde in den Töpfen in einer Tiefe von 1 cm gestreut.

Nach der Einsaat wurden die Pflanzen 14 Tage angezogen, und eine Test-Chemikalie in einer festgelegten Konzentration, hergestellt wie im vorerwähnten Beispiel, wurde gleichmäßig über die Test-Pflanzen in jedem der Test-Töpfe gesprüht.

Vier Wochen nach dem Sprühen wurden die herbizide Wirkung und die Phytotoxizität gegenüber den Nutzpflanzen anhand der gleichen Bewertungsskalen wie im vorhergehenden Beispiel geprüft.

Test-Ergebnisse der Laub-Behandlung

| Aktive Verbindung | Dosis kg/ha | Herbizider Effekt AMALI | CHEAL | Phytotoxischer Effekt ORYSD | GLXMA | ZEAMX |
|---|---|---|---|---|---|---|
| Verbindung der Formel (I) der vorliegenden Erfindung | 0,06 | 5 | 5 | 1 | 2 | 1 |
| bekannte Verbindung E-1 | 0,25 | 4 | 4 | 3 | 3 | 2 |

Anmerkung:

Die in der Tabelle verwendeten Abkürzugen für die Un kräuter und Nutzpflanzen sind die gleichen wie in der Tabelle des vorhergehenden Beispiels.

Wie in dem im hiernach beschriebenen Bezugsbeispiel aufgezeigt wird, ist das Benzoxazin der Formel (I) der vorliegenden Erfindung auch als neues Zwischenprodukt für die Herstellung des herbizid aktiven 2-[4-Cyanomethyl-7-fluoro-2H-1,4-benzoxazin-3(4H)-on-6-yl]-4,5,6,7-tetrahydro-2H-isoindol-1,3-dions der folgenden Formel

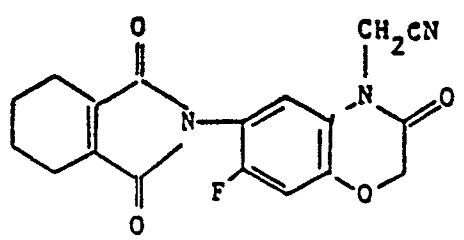

(E)

verwendbar.

Bezugsbeispiel E

Herstellungsbeispiel für die oben erwähnte herbizide Verbindung der Formel (E)]

N,N-Dimethylformamid (3 Tropfen) wurde zu einer Mischung bestehend aus 2-[4-Carbamoylmethyl-7-fluoro-2H-1,4-benzoxazin-3(4H)-on-6-yl]-4,5,6,7-tetrahydro-2H-isoindol-1,3-dion (0,2 g), Toluol (30 ml) und Thionylchlorid (0,2 g) hinzugefügt, und dann wurde die Mischung 3 h zum Rückfluß erhitzt. Nach dem

Konzentrieren der Lösung wurde Toluol (50 ml) zugegeben, das Produkt wurde gut durchgemischt, und dann wurde das Toluol abdestilliert. Der resultierende feste Stoff wurde aus Ethanol umkristallisiert, wonach das gewünschte 2-[4-Cyanomethyl-7-fluoro-2H-1,4-benzoxazin-3(4H)-on-6-yl]-4,5,6,7-tetrahydro-2H-isoindol-1,3-dion (0,15 g) erhalten wurde; Schmp. 225-229 °C.

**Ansprüche**

1. 2-[4-Carbamoylmethyl-7-fluoro-2H-1,4-benzoxazin-3(4H)-on-6-yl]-4,5,6,7-tetrahydro-2H-isoindol-1,3-dion der Formel (I)

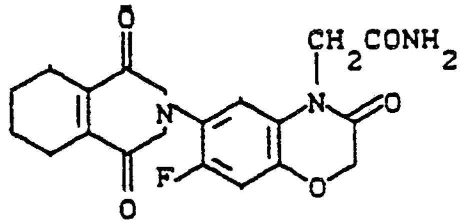

(I)

2. Verfahren zur Herstellung der Verbindung der Formel (I)

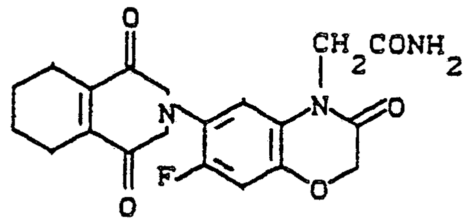

(I)

dadurch gekennzeichnet, daß
4-Carbamoylmethyl-7-fluoro-6-amino-2H-1,4-benzoxazin-3(4H)-on der Formel (II)

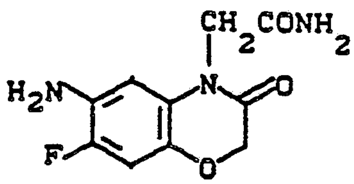

(II)

mit 3,4,5,6-Tetrahydrophthalsäureanhydrid in Gegenwart inerter Lösungsmittel umgesetzt wird.

3. Herbizide Zusammensetzungen, dadurch gekennzeichnet, daß sie 2-[4-Carbamoylmethyl-7-fluoro-2H-1,4-benzoxazin-3(4H)-on-6-yl]-4,5,6,7-tetrahydro-2H-isoindol-1,3-dion der Formel (I) nach den Ansprüchen 1 und 2 enthalten.

4. Verfahren zur Unkrautbekämpfung, dadurch gekennzeichnet, daß man 2-[4-Carbamoylmethyl-7-fluoro-2H-1,4-benzoxazin-3(4H)-on-6-yl]-4,5,6,7-tetrahydro-2H-isoindol-1,3-dion der Formel (I) nach den Ansprüchen 1 und 2 auf Unkräuter und/oder ihren Lebensraum einwirken läßt.

5. Verwendung von 2-[4-Carbamoylmethyl-7-fluoro-2H-1,4-benzoxazin-3(4H)-on-6-yl]-4,5,6,7-tetrahydro-2H-isoindol-1,3-dion der Formel (I) nach den Ansprüchen 1 und 2 zur Unkrautbekämpfung.

6. Verfahren zur Herstellung herbizider Zusammensetzungen, dadurch gekennzeichnet, daß 2-[4-Carbamoylmethyl-7-fluoro-2H-1,4-benzoxazin-3(4H)-on-6-yl]-4,5,6,7-tetrahydro-2H-isoindol-1,3-dion der Formel (I) nach den Ansprüchen 1 und 2 mit Streckmitteln und/oder grenzflächenaktiven Mitteln vermischt wird.

7. Verbindungen der folgenden Formel

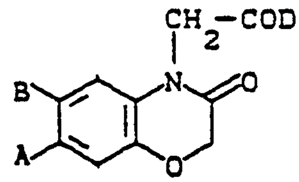

,

in der
A Wasserstoff oder Fluor ist,
B Wasserstoff oder Nitro ist und
D Amino, Hydroxy oder Ethoxy ist.

8. Verbindungen nach Anspruch 7, nämlich 4-Carbamoylmethyl-7-fluoro-6-nitro-2H-1,4-benzoxazin-3(4)-on

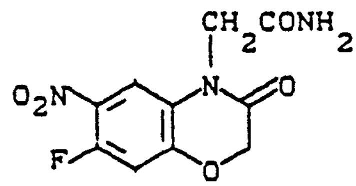

,

4-Carbamoylmethyl-7-fluoro-2H-1,4-benzoxazin-3(4H)-on

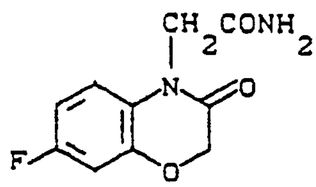

,

4-Carboxymethyl-7-fluoro-2H-1,4-benzoxazin-3(4H)-on

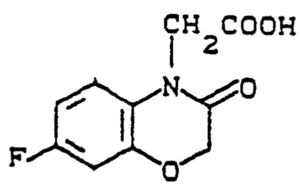

,

4-Carboxymethyl-7-fluoro-6-nitro-2H-1,4-benzoxazin-3(4)-on

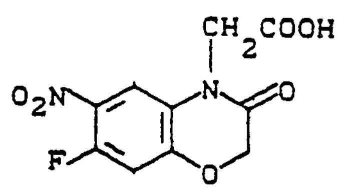

,

4-Ethoxycarbonylmethyl-7-fluoro-6-nitro-2H-1,4-benzoxazin-3(4H)-on

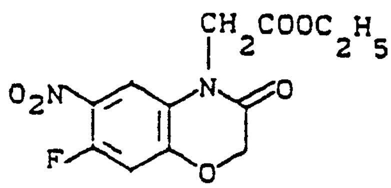

,

und
4-Ethoxycarbonylmethyl-7-fluoro-2H-1,4-benzoxazin-3(4H)-on